(19) Europäisches Patentamt
European Patent Office
Office européen des brevets

(11) Numéro de publication: **0 008 962**
**B1**

# (12) FASCICULE DE BREVET EUROPEEN

(45) Date de publication du fascicule du brevet: **09.02.83**

(51) Int. Cl.³: **C 07 D 401/06**, A 61 K 31/47

(21) Numéro de dépôt: **79400479.6**

(22) Date de dépôt: **10.07.79**

(54) **Nouveaux dérivés de la ((quinolyl-4)-3 propyl-1)-4 pipéridine et médicaments les contenant.**

(30) Priorité: **03.08.78 FR 7822968**

(43) Date de publication de la demande:
**19.03.80 Bulletin 80/6**

(45) Mention de la délivrance du brevet:
**09.02.83 Bulletin 83/6**

(84) Etats contractants désignés:
**BE CH DE FR GB IT LU NL SE**

(56) Documents cités:
**EP - A - 0 005 654**
**DE - A - 2 727 144**
**FR - A - 2 354 771**

(73) Titulaire: **PHARMINDUSTRIE**
**35 Quai du Moulin de Cage**
**F-92231 Gennevilliers (FR)**

(72) Inventeur: **Champseix, Alain André**
**12, rue Hector Berlioz**
**Forges les Bains F-91470 Limours (FR)**
Inventeur: **Gueremy, Claude Georges Alexandre**
**3, rue Daumesnil**
**F-78800 Houilles (FR)**
Inventeur: **Le Fur, Gérard Roger**
**17, rue du 11 Novembre**
**F-92390 Villeneuve La Garenne (FR)**

(74) Mandataire: **Houssin, Jean et al,**
**PRODUITS CHIMIQUES UGINE KUHLMANN**
**Service Propriété Industrielle Tour Manhattan**
**Cedex 21**
**F-92087 Paris La Defense 2 (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

EP 0 008 962 B1

Nouveaux dérivés de la [(quinolyl-4)-3 propyl-1]-4 pipéridine et médicaments les contenant

Dans la demande de brevet français n° 76 18555 déposée le 18 Juin 1976 et publiée sous le n° 2 354 771 ont été décrits des médicaments contenant en tant que principe actif un dérivé de la [(quinolyl-4)-3 propyl-1]-4 pipéridine répondant à la formule générale:

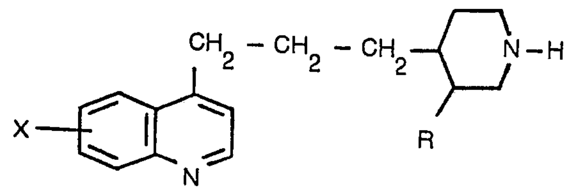

(I)

dans laquelle ou bien X et R sont tous les deux des atomes d'hydrogène, ou bien R est un groupe vinyle ou éthyle et X est un atome d'hydrogène ou un groupe méthoxy en position 6, les atomes de carbone du cycle pipéridinique porteurs du groupement R et du groupement (quinolyl-4)-3 propyl ayant alors tous les deux la configuration rectus (R).

La présente invention a pour objet des médicaments contenant en tant que principe actif un dérivé de la [(quinolyl-4)-3 propyl-1]-4 pipéridine répondant à la formule:

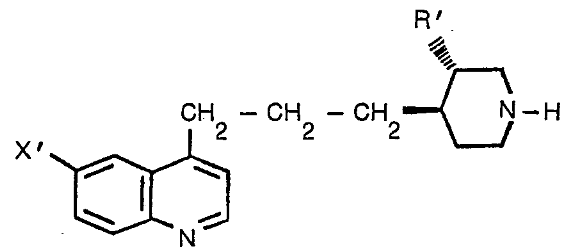

(II)

dans laquelle X′ est un atome d'hydrogène ou un groupe méthoxy, R′ est un groupe vinyle ou éthyle, le carbone portant le groupement R′ a la configuration sinister (S) et le carbone portant le groupement (quinolyl-4)-3 propyl, c'est-à-dire le carbone en position 4 sur le cycle de la pipéridine, a la configuration rectus (R), ou un sel d'un tel dérivé avec un acide pharmaceutiquement acceptable.

Le composé de formule (II) pour lequel X′ est un groupe méthoxy et R′ est un groupe vinyle a déjà été décrit dans la demande de brevet européen E — A — 0 005 654 publiée le 28 Novembre 1979 et bénéficiant d'une date de priorité antérieure au 3 Août 1978. Les composés de formule (II) pour lesquels ou bien X′ est un atome d'hydrogène et R′ est un groupe vinyle ou éthyle, ou bien X′ est un groupe méthoxy et R′ est un groupe éthyle sont nouveaux et font partie en tant que tels de l'invention.

Les composés de formule (II) peuvent être préparés par réduction, notamment au moyen de l'hydrate d'hydrazine en présence d'un hydroxyde de métal alcalin et d'un solvant tel qu'un alcool, des composés de formule:

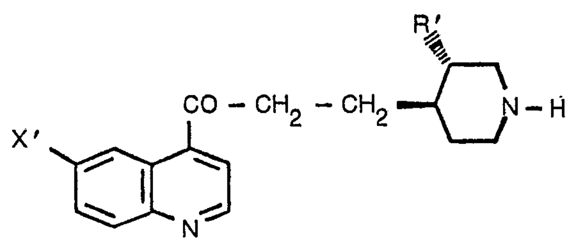

(III)

dans laquelle X′ et R′ ont la même signification que dans la formule (II), le carbone porteur du groupement R′ a la configuration sinister (S) et le carbone en position 4 sur le cycle de la pipéridine a la configuration rectus (R).

Les composés de formule (III) pour lesquels R′ est un groupe vinyle peuvent eux-mêmes être préparés par chauffage en milieu acide des composés de formule:

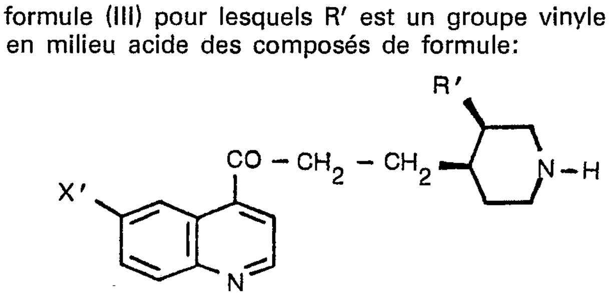

(III bis)

dans laquelle R′ est un groupe vinyle et le carbone porteur du groupement R′ et celui en position 4 sur le cycle de la pipéridine ont tous les deux la configuration rectus (R).

Les composés de formule (II) pour lesquels R′ est un groupe éthyle peuvent également être préparés par hydrogénation catalytique des composés correspondants de formule (II) pour lesquels R′ est un groupe vinyle. Cette hydrogénation peut par exemple être réalisée à température ambiante, sous une pression d'hydrogène égale à la pression atmosphérique, au sein d'un solvant inerte tel qu'un alcool (par exemple le méthanol ou l'éthanol) ou un acide (par exemple l'acide acétique), en présence d'un catalyseur tel que le palladium, le nickel, le rhodium, le ruthénium ou le platine.

Enfin les composés de formule (II) pour lesquels R′ est un groupe vinyle peuvent également être préparés par chauffage en milieu acide des composés de formule:

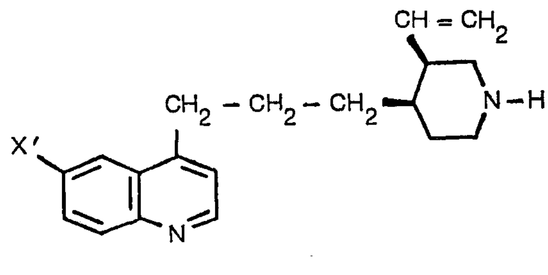

(IV)

dans laquelle le carbone porteur du groupe vinyle et celui en position 4 sur le cycle de la pipéridine ont la configuration rectus (R).

Une fois la réaction terminée, le mélange réactionnel obtenu dans les procédés ci-dessus est traité suivant des méthodes classiques, physiques (évaporation, extraction à l'aide d'un solvant, distillation, cristallisation, chromatographie, etc. . .) ou chimiques (formation de sel et régénération de la base, etc. . .) afin d'isoler les produits de formule (II) à l'état pur, soit sous forme de la base libre, soit sous forme d'un sel de celle-ci avec un acide.

Les composés de formule (II) sous forme de base libre peuvent éventuellement être transformés en sels d'addition avec un acide minéral ou organique par action d'un tel acide au sein d'un solvant approprié.

Comme il a été indiqué dans la demande de brevet français n° 76 18555, les composés de formule (I) sont utiles pour le traitement des états pathologiques engendrés par une perturbation du fonctionnement des systèmes sérotoninergiques et trouvent en particulier des applications comme agents psychotropes, plus particulièrement comme agents antidépresseurs. Ces applications sont en relation avec la propriété d'inhiber la recapture de la sérotonine par les membranes des neurones cérébraux que possèdent les composés de formule (I).

Les composés de formule (I) possèdent également la propriété de provoquer la libération de la sérotonine contenue soit dans les neurones, soit dans les plaquettes sanguines.

Les composés de formule (II) possèdent les deux propriétés signalées ci-dessus pour les composés de formule (I), mais chez eux la propriété de provoquer la libération de la sérotonine est considérablement plus marquée que celle d'inhiber la recapture de cette amine. Ceci pourrait se traduire en thérapeutique par une action très rapide lors du traitement des états de dépression (dans ce cas le produit agit sur la sérotonine des neurones cérébraux) et lors du traitement des migraines (dans ce cas le produit agit sur la sérotonine plaquettaire).

Les exemples suivants illustrent la préparation des composés de formule (II).

Exemple 1

[(méthoxy-6 quinolyl-4)-3 propyl-1]-4 (R) vinyl-3 (S) pipéridine.

A une suspension de 1,1 g de dichlorhydrate de (méthoxy-6 quinolyl-4)-1 [vinyl-3 (S) pipéridyl- 4 (R)]-3 propanone-1 dans 3,5 ml de diéthylène-glycol et 0,18 ml d'une solution aqueuse à 85% d'hydrate d'hydrazine, on ajoute 0,31 g d'hydroxyde de potassium. On chauffe lentement jusqu'à 150°C puis on refroidit à 100°C et on introduit 0,47 g d'hydroxyde de potassium. On porte le mélange réactionnel à 150°C et on le maintient à cette température pendant 5 heures.

Après refroidissement le milieu réactionnel est traité par 15 ml d'eau. L'huile qui relargue est extraite par de l'acétate d'éthyle. La phase organique est décantée, lavée, séchée sur du sulfate de magnésium puis évaporée. L'huile brute obtenue est fixée sur une colonne contenant 45 g de silice et on élue ensuite avec un mélange contenant 90% de chloroforme et 10% de diéthylamine.

Le produit purifié ainsi isolé est mis en solution dans l'acétone et transformé en chlorhydrate par addition d'une solution d'acide chlorhydrique dans l'éther. On obtient 0,24 g de chlorhydrate de [(méthoxy-6 quinolyl-4)-3 propyl-1]-4 (R) vinyl-3 (S) pipéridine, qui fond à 151°C.

La cétone de départ peut être préparée comme suit:

A 2,1 g de (méthoxy-6 quinolyl-4)-1 [vinyl-3 (R) pipéridyl-4 (R)]-3 propanone-1 (quinicine) on ajoute 20 ml d'eau distillée et on amène le pH à 3,5 par addition d'une solution N d'acide sulfurique. On introduit ce mélange dans un autoclave en acier inoxydable de 225 ml et on chauffe 48 h à 140°C. On alcalinise

ensuite la solution par addition d'une solution 2 N d'hydroxyde de sodium et on extrait avec de l'éther. L'extrait éthéré est lavé avec de l'eau, séché sur du sulfate de sodium anhydre et évaporé à sec.

Le résidu obtenu (1,7 g) est dissous dans un peu d'un mélange toluène-diéthylamine 9/1 et fixé sur une colonne contenant 500 g de silice. On élue ensuite avec un mélange toluène-diéthylamine 9/1, sous une pression de 4 bars. On isole ainsi 0,51 g du produit de départ (quinicine) et 1,08 g de (méthoxy-6 quinolyl-4)-1 [vinyl-3 (S) pipéridyl-4 (R)]-3 propanone-1. Ce dernier composé est mis en solution dans le méthanol et transformé en chlorhydrate par addition d'une solution 8 N d'acide chlorhydrique dans le méthanol.

Exemple 2

[(méthoxy-6 quinolyl-4)-3 propyl-1]-4 (R) vinyl-3 (S) pipéridine.

2,1 g de [(méthoxy-6 quinolyl-4)-3 propyl-1]-4 (R) vinyl-3 (R) pipéridine sont dissous dans 20 ml d'eau distillée. On ajuste le pH à 2 par addition d'une solution 5 N d'acide sulfurique. On introduit ce mélange dans un autoclave en acier inox de 225 ml et on chauffe 48 h à 140°C. On alcalinise ensuite la solution par addition d'une solution 2 N d'hydroxyde de sodium et on extrait avec de l'éther. L'extrait éthéré est lavé avec de l'eau, séché sur du sulfate de sodium anhydre et évaporé à sec. Le résidu obtenu (1,9 g) est absorbé sur une colonne de silice de 500 g. Par élution avec un mélange toluène-diéthylamine 9/1 sous une pression d'élution de 4 bars, on isole 0,71 g de produit de départ et 0,68 g de [(méthoxy-6 quinolyl-4)-3 propyl-1]-4 (R) vinyl-3 (S) pipéridine sous forme d'une huile.

Cette huile est mise en solution dans le méthanol et le produit ci-dessus est transformé en son chlorhydrate par addition d'une solution d'acide chlorhydrique dans le méthanol.

Caractéristiques du chlorhydrate de [(méthoxy-6 quinolyl-4)-3 propyl-1]-4 (R) vinyl-3 (S) pipéridine:

Point de fusion: 151°C

Pouvoir rotatoire (mesuré dans l'eau): $[\alpha]_D^{25} = -31°$

Spectre de résonance magnétique nucléaire (solvant: deutérochloroforme; référence: tétraméthylsilane):

Les déplacements chimiques $\delta$ des protons numérotés 10, 11 et 11′ dans la formule (V) ci-dessous sont:

$$\delta_{10} = 5{,}4 \text{ ppm}$$
$$\delta_{11,\,11'} = 5 \text{ ppm}$$

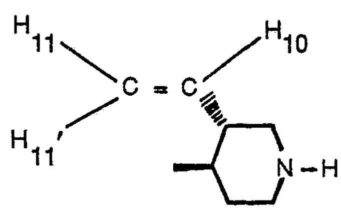

(V)

La préparation de la [(méthoxy-6 quinolyl-4)-3 propyl-1]-4 (R) vinyl-3 (R) pipéridine utilisée comme produit de départ est décrite dans la demande française n° 76 18555.

Exemple 3:

[(méthoxy-6 quinolyl-4)-3 propyl-1]-4 (R) éthyl-3 (S) pipéridine.

Une suspension bien agitée de 2 g de charbon palladié à 10% de palladium dans une solution de 6,8 g de monochlorhydrate de [(méthoxy-6 quinolyl-4)-3 propyl-1]-4 (R) vinyl-3 (S) pipéridine dans 100 ml d'éthanol absolu est maintenue à la température ambiante sous une pression d'hydrogène correspondant à une surpression de 50 mm d'eau par rapport à la pression atmosphérique jusqu'à cessation de l'absorption de l'hydrogène.

Le charbon palladié est ensuite séparé par filtration puis la solution alcoolique est concentrée. Le résidu est dissous dans 50 ml d'eau et la solution obtenue est amenée à pH = 10 par addition d'une solution d'hydroxyde de sodium.

L'huile qui relargue est extraite par du chloroforme et l'extrait est lavé à l'eau puis séché sur du sulfate de magnésium. Après évaporation du chloroforme, l'huile résiduelle (5,6 g) est transformée en fumarate par dissolution dans l'éthanol et addition de 2,1 g d'acide fumarique.

On obtient 5,5 g de fumarate acide de [(méthoxy-6 quinolyl-4)-3 propyl-1]-4 (R) éthyl-3 (S) pipéridine fondant à 180°C.

Analyse pour $C_{20}H_{28}N_2O$, $C_4H_4O_4$

| | %N |
|---|---|
| *Calculé* ...................... | 6,54 |
| *Trouvé* ...................... | 6,47 |

Exemple 4:

[(quinolyl-4)-3 propyl-1]-4 (R) vinyl-3 (S) pipéridine.

En opérant comme à l'exemple 2 mais en remplaçant au départ les 2,1 g de [(méthoxy-6 quinolyl-4)-3 propyl-1]-4 (R) vinyl-3 (R) pipéridine par 1,3 g de [(quinolyl-4)-3 propyl-1]-4 (R) vinyl-3 (R) pipéridine, on obtient 0,8 g de [(quinolyl-4)-3 propyl-1]-4 (R) vinyl-3 (S) pipéridine sous forme d'une huile.

Caractéristiques de la [(quinolyl-4)-3 propyl-1]-4 (R) vinyl-3 (S) pipéridine:

Spectre de R.M.N.:

déplacements chimiques des protons numérotés 10, 11, et 11' dans la formule (V):

$$\delta_{10} = 5{,}4 \text{ ppm}$$
$$\delta_{11.11'} = 5{,}05 \text{ ppm}$$

La préparation de la [(quinolyl-4)-3 propyl-1]-4 (R) vinyl-3 (R) pipéridine utilisés comme produit de départ est décrite dans la demande française n° 76 18555.

PROPRIETES PHARMACOLOGIQUES

On sait que la recapture de la sérotonine par les plaquettes sanguines constitue un bon modèle de la recapture de cette amine par les neurones (cf. J. TUOMISTO, J. Pharm., Pharmac., *26*, 92 (1974). Appliquée à l'étude des médicaments, une méthode mettant en oeuvre les plaquettes sanguines présente le grand intérêt de permettre l'utilisation de cellules humaines, ce qui lui confère un bon caractère prévisionnel.

La capacité des produits à inhiber la recapture de la sérotonine ou à provoquer sa libération a été mise en évidence sur des plaquettes sanguines humaines, selon J. L. DAVID et Coll. "Platelets Function and thrombosis, a review of methods" p. 335 (Plenum Press, LONDON 1972).

1) *Inhibition de la recapture de la sérotonine*

Les résultats sont exprimés par une dose inhibitrice 50% ou $I_{50}$, qui représente la dose de produit, en micromoles par litre, diminuant de 50% la recapture de la sérotonine.

2) *Libération de la sérotonine*

L'action des produits sur la libération de la sérotonine est testée à deux concentrations: $5 \times 10^{-6}$ mole/litre et $5 \times 10^{-5}$ mole/litre.

Les résultats obtenus sont exprimés en pourcentage d'augmentation de la libération de la sérotonine par rapport aux résultats fournis par les témoins.

Les résultats obtenus sont rassemblés dans le tableau ci-après. Dans ce tableau figurent à titre comparatif, les résultats obtenus avec deux produits de référence (imipramine et p-chloro amphétamine).

TABLEAU

| Produit | Inhibition de la recapture de la sérotonine $I_{50}$ en micromoles par litre | Pourcentage d'augmentation de la libération de la sérotonine | |
|---|---|---|---|
| | | Conc. du produit $5 \times 10^{-6}$ mole/litre | Conc. du produit $5 \times 10^{-5}$ mole/litre |
| Composé de l'exemple 1 | 1 | 40 | 78 |
| Composé de l'exemple 3 | >0,1 | 30 | 69 |
| [(méthoxy-6 quinolyl-4)-3 propyl-1]-4(R)vinyl-3(R) pipéridine, épimère du composé de l'exemple 1 | 0,01 | 7 | 32 |
| [(méthoxy-6 quinolyl-4)-3 propyl-1]-4(R) éthyl-3(R) pipéridine, épimère du composé de l'exemple 3 | 0,01 | 34 | 67 |
| Imipramine | 0,4 | 3 | 13 |
| p-chloro-amphétamine | 12 | 6 | 51 |

Il ressort du tableau que les composés des exemples 1 et 3 sont beaucoup moins actifs que leurs épimères comme inhibiteurs de recapture de la sérotonine (cent fois moins actif pour le composé de l'exemple 1, au moins 10 fois moins actif pour le composé de l'exemple 3). Par contre les composés des exemples 1 et 3 sont de puissants agents de libération de la sérotonine, encore plus puissants que la p-chloro amphétamine.

PROPRIETES TOXICOLOGIQUES

La toxicité aiguë des produits a été déterminée chez la souris mâle $CD_1$ (Charles RIVER) par voie orale.

La $DL_{50}$ calculée, après 3 jours d'observation, par la méthode cumulative de J. J. REED at Coll. (Am. J. Hyg. *27*, 493—1938) est de 225 mg/kg pour le composé de l'exemple 1 et d'environ 200 mg/kg pour le composé de l'exemple 3.

Les composés de formule (II) sont atoxiques à 100 mg/kg et se comportent donc comme des substances relativement peu toxiques chez la souris.

UTILISATION THERAPEUTIQUE

Les composés de formule (II) et leurs sels pharmaceutiquement acceptables peuvent être utilisés en thérapeutique humaine sous forme de comprimés, capsules, gélules, suppositoires, solutions ingérables ou injectables, etc... comme régulateurs du tonus vasculaire sérotonino-dépendant, notamment pour le traitement des migraines, et comme médicaments thymoanaleptiques à action particulièrement rapide (à cause de leur action sur la libération de la sérotonine).

La posologie dépend des effets recherchés et de la voie d'administration utilisée. Par exemple, par voie orale, elle peut être comprise entre 15 et 250 mg de substance active par jour, avec des doses unitaires allant de 5 à 50 mg.

**Revendications**

1. Les composés de formule:

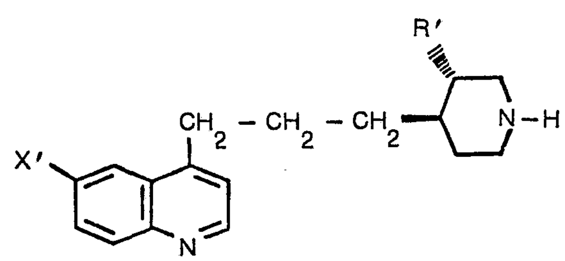

dans laquelle X' est un atome d'hydrogène ou un groupe méthoxy, R' est un groupe éthyle et peut en outre être, lorsque X' est un atome d'hydrogène, un groupe vinyle, le carbone portant le groupement R' a la configuration sinister (S) et celui portant le groupement (quinolyl-4)-3 propyl a la configuration rectus (R), et leurs sels d'addition avec les acides.

2. Médicament pour le traitement des états de dépression et des migraines caractérisé en ce qu'il contient comme agent actif un composé répondant à la formule:

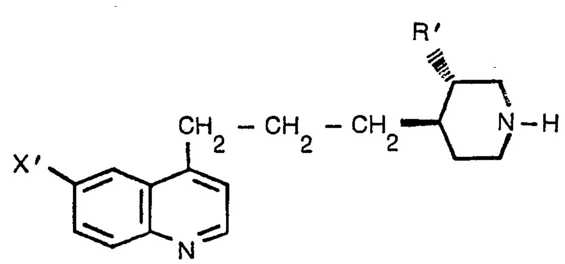

dans laquelle X' est un atome d'hydrogène ou un groupe méthoxy, R' est un groupe vinyle ou éthyle, le carbone portant le groupement R' a la configuration sinister (S) et celui portant le groupement (quinolyl-4)-3 propyl a la configuration rectus (R), ou un sel d'un tel composé avec un acide pharmaceutiquement acceptable.

**Claims**

1. Compounds of the formula:

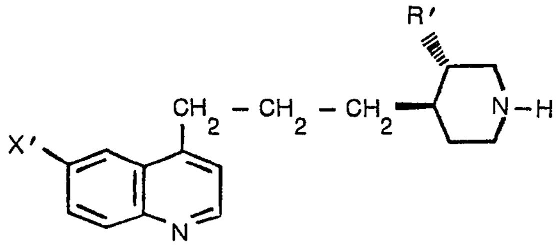

in which X′ is a hydrogen atom or a methoxy group, R′ is an ethyl group and may also be, when X′ is a hydrogen atom, a vinyl group, the carbon carrying the R′ group having a sinister configuration (S) and that carrying the 3-(4-quinolyl) propyl group having a rectus configuration (R), and their addition salts with acids.

2. Medicament for the treatment of depressive states and migrains characterised in that it contains as the active agent, a compound corresponding to the formula:

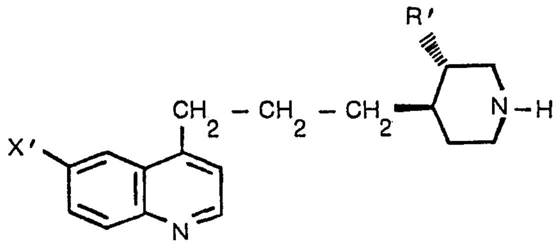

in which X′ is a hydrogen atom or a methoxy group, R′ is a vinyl or ethyl group, the carbon carrying the R′ group having a sinister configuration (S) and that carrying the 3-(4-quinolyl) propyl group having a rectus configuration (R), or a salt of such a compound with a pharmaceutically acceptable acid.

**Patentansprüche**

1. Verbindungen der Formel

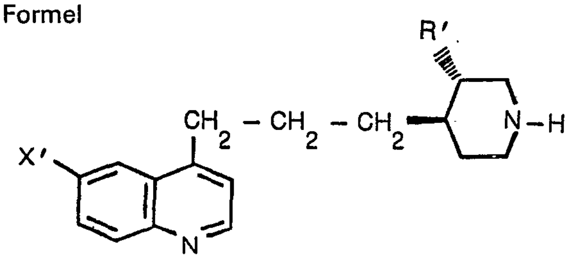

worin X′ ein Wasserstoffatom oder eine Methoxygruppe, R′ eine Äthylgruppe oder ausserdem, wenn die Gruppe X′ ein Wasserstoffatom darstellt, eine Vinylgruppe bedeuten, wobei das die Gruppe R′ tragende Kohlenstoffatom die Konfiguration S (sinister) und das die 3-(4-Chinolyl)-propylgruppe tragende Kohlenstoffatom die Konfiguration R (rectus) aufweist, sowie deren Additionssalze mit Säuren.

2. Arzneimittel zur Behandlung von Depressionszuständen und Migränen, dadurch gekennzeichnet, daß es als Wirkstoff eine Verbindung der Formel

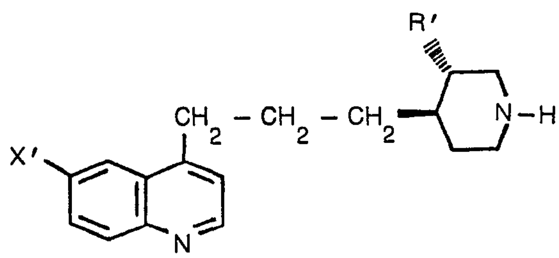

worin X′ ein Wasserstoffatom oder eine Methoxygruppe, R′ eine Äthylgruppe oder eine Vinylgruppe bedeuten, wobei das die Gruppe R′ tragende Kohlenstoffatom die Konfiguration S (sinister) und das die 3-(4-Chinolyl)-propylgruppe tragende Kohlenstoffatom die Konfiguration R (rectus) aufweist, oder ein Salz einer solchen Verbindung mit einer pharmazeutisch annehmbaren Säure enthält.